# EUROPÄISCHE PATENTANMELDUNG

(11) **EP 3 115 069 A1**
(43) Veröffentlichungstag der Anmeldung: **11.01.2017**
(21) Anmeldenummer: 15175768.9
(22) Anmeldetag: 07.07.2015
(51) Int. Cl.: A61M 1/10

(54) **VORRICHTUNG ZUR POSITIONSBESTIMMUNG EINES BEWEGLICHEN BAUTEILS**

(71) Anmelder: Berlin Heart GmbH, 12247 Berlin (DE)
(72) Erfinder: GRASSE, Martin, 10317 Berlin (DE); KRAMBECK, Helge, 12163 Berlin (DE)
(74) Vertreter: Pfenning, Meinig & Partner mbB

(57) **Zusammenfassung**

Vorrichtung zur Positionsbestimmung eines zumindest in einer Richtungsachse (300) beweglichen Bauteils (400) einer Blutpumpe mit zwei von einem Wechselstrom durchflossenen Sensorspulen (100, 200), deren magnetische Felder durch das bewegliche Bauteil (400) beeinflusst werden, wobei die Sensorspulen (100, 200) in einer Brückenschaltung angeordnet sind, die ausgebildet ist, ein von der Differenz der Wechselstromwiderstände der Sensorspulen (100, 200) abhängiges Positionssignal auszugeben.

## Beschreibung

Die Erfindung betrifft eine Vorrichtung und eine Auswerteschaltung zur Erzeugung eines Positionssignales mittels eines induktiven Lagesensors oder mehrerer induktiver Lagesensoren zur Bestimmung der Position eines Bauteils.

Insbesondere betrifft die Erfindung ein Verfahren und eine Auswerteschaltung zur Bestimmung der Position eines magnetisch gelagerten rotierenden Bauteils, z. B. des Rotors eines bürstenlosen Synchronmotors, für dessen Lageregelung. Die Lageregelung beeinflusst in Abhängigkeit vom Positionssignal das Magnetfeld der magnetischen Lagerung mit Hilfe von Steuerstromspulen. Der Synchronmotor kann beispielsweise als Antrieb für eine axiale oder radiale Fluidförderpumpe dienen.

Blut stellt ein besonders empfindliches Fluid dar und ist im natürlichen Kreislaufsystem hermetisch von der Umwelt abgeschirmt, so dass es keinen Fremdeinflüssen unterliegt. Besteht jedoch die Notwendigkeit, das Herz durch eine künstliche Blutpumpe zu ersetzen oder den Kreislauf durch eine zusätzliche Herzpumpe zu unterstützen, so kommt es zu Wechselwirkungen des Blutes mit dem technischen System. Das Blut unterliegt dann leicht der Hämolyse oder Thrombenbildung, mit den entsprechenden nachteiligen Wirkungen für den Patienten. Es sind deshalb in jüngster Zeit verstärkt Anstrengungen unternommen worden, um Fluidförderpumpen so auszubilden, dass es zu möglichst wenig mechanischen Einflüssen auf das Blut bzw. auf andere empfindliche Fluide kommt. Eine Möglichkeit hierzu ist die magnetische Lagerung des rotierenden Elements eines Pumpenantriebes.

Eine solche Fluidpumpe lässt sich üblicherweise in einen bürstenlosen Synchronmotor integrieren. Sie besteht nach der WO 00/64030 im Wesentlichen aus einem zylindrischen Rohr, das beidseitig mit dem Fluidsystem verbunden werden kann. Das Rohr wird von dem Stator, bestehend aus Blechpaket, Wicklung und Eisenrückschlusskappen, umgeben. Der Rotor enthält permanentmagnetische Felderreger und weist an seinem Außenmantel Fördereinrichtungen für das Fluid auf, so dass dieses im Ringraum zwischen dem Rohr und dem Rotor axial gefördert wird.

Der Rotor wird magnetisch gelagert. Er trägt zu diesem Zweck an seinen beiden Stirnseiten angebrachte Permanentmagnete, die in axialer Richtung magnetisiert sind. Den Permanentmagneten des Rotors stehen gegensinnig magnetisierte Permanentmagnete gegenüber, die z. B. am Rotorgehäuse angeordnet sein können.

In radialer Richtung wirken die auf gegenseitige Anziehung orientierten Magnetfelder stabilisierend, das heißt, die radiale Lagerung ist passiv stabil. In axialer Richtung kann der Rotor von einem der beiden Permanentmagnetpaare stärker angezogen werden. Um die Lage des Rotors in axialer Richtung zu stabilisieren, werden beispielsweise ein oder mehrere Steuerspulen an den Statorseiten so angeordnet, dass ein Stromfluss durch die Steuerspule das Magnetfeld des einen Permanentmagnetpaares abschwächt und das des anderen verstärkt. Der Steuerstrom wird in Abhängigkeit von der aktuellen axialen Rotorposition geregelt.

Anstelle von Permanentmagneten können für die Rotorlagerung im Stator und im Rotor auch Elektromagnete verwendet werden. Alternativ können anstelle der Permanentmagnete für die Rotorlagerung im Stator Elektromagnete und im Rotor Weicheisenelemente verwendet werden. Bei Verwendung von Elektromagneten können die bei Permanentmagneten erforderlichen zusätzlichen Steuerspulen entfallen. Die axiale Lageregelung des Rotors erfolgt dann über die Steuerung des Stromflusses durch die Elektromagneten der Lagerung. Mit mehreren, beispielsweise radial angeordneten Elektromagneten kann zusätzlich zur axialen auch die radiale Rotorlage gesteuert werden.

Für die Lageregelung des Rotors muss die Rotorposition mit Hilfe eines oder mehrerer Lagesensoren bestimmt werden. Geeignete Lagesensoren sind beispielsweise induktive Lagesensoren.

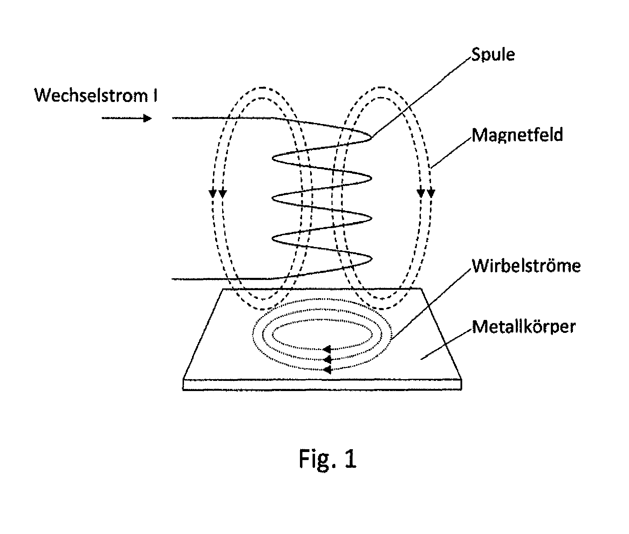

Ein induktiver Lagesensor umfasst eine Sensorspule. Gegenüber der Sensorspule ist ein dem beweglichen Bauteil verbundener leitfähiger Metallkörper, beispielsweise ein Aluminiumkörper, derart angeordnet, dass er sich im magnetischen Feld der Sensorspule befindet. Wird, wie in Fig. 1 schematisch dargestellt, die Sensorspule von einem Wechselstrom I durchflossen, induziert das in Fig. 1 durch gestrichelte Linien symbolisierte magnetische Feld der Sensorspule in dem leitfähigen Metallkörper Wirbelströme, die in Fig. 1 durch gepunktete Linien symbolisiert werden. Da die Stärke der Wirbelströme abhängig vom Abstand des Metallkörpers zur Sensorspule ist, bewirkt eine Annäherung des mit dem beweglichen Bauteil verbundenen leitfähigen Metallkörpers eine Vergrößerung des Wechselstromwiderstandes der Sensorspule, wohingegen sich der Wechselstromwiderstand der Sensorspule im umgekehrten Fall verkleinert. Aus dem Wechselstromwiderstand der Sensorspule kann so ein Signal als Messgröße für den Abstand des beweglichen Bauteils zur Sensorspule und damit für dessen Position bestimmt werden.

Nachteilig ist dabei der Aufwand für die Messung des Wechselstromwiderstandes. Es ist außerdem wünschenswert, eine hohe Genauigkeit der Positionsmessung schon bei kleinen Positionsänderungen zu erreichen. Weiterhin ist der Wechselstromwiderstand der Sensorspule temperaturabhängig, so dass Temperaturänderungen der Sensorspule die Positionsmessung verfälschen können.

Der Erfindung liegt damit die Aufgabe zugrunde, eine Vorrichtung für die Positionsbestimmung eines beweglichen Bauteils zu schaffen, die diese Nachteile verringert.

Erfindungsgemäß wird die Aufgabe durch die Merkmale der Patentansprüche 1 oder 15 gelöst. Zweckmäßige Ausgestaltungen sind Gegenstand der Unteransprüche.

Danach umfasst eine Vorrichtung zur Positionsbestimmung eines zumindest in einer Richtungsachse beweglichen Bauteils einer Blutpumpe zwei von einem Wechselstrom durchflossene Sensorspulen, deren magnetischen Felder durch das bewegliche Bauteil beeinflusst werden, wobei die Sensorspulen in einer Brückenschaltung angeordnet sind, die ausgebildet ist, ein von der Differenz der Wechselstromwiderstände der Sensorspulen abhängiges Positionssignal auszugeben. Dadurch kann in vorteilhafter Art und Weise eine höhere Genauigkeit der Positionsbestimmung erreicht werden.

Das Bauteil der Blutpumpe kann beispielsweise durch den bereits erwähnten, in axialer Richtung beweglichen, magnetisch berührungslos gelagerten Rotor der Blutpumpe gebildet werden.

Die Brückenschaltung ist ausgebildet, den Realteil oder den Imaginärteil des komplexen Wechselstromwiderstandes der Sensorspulen auszuwerten.

Beispielsweise wird die Brückenschaltung durch die Parallelschaltung einer ersten Reihenschaltung aus einer ersten Sensorspule mit einem ersten Widerstand und einer zweiten Reihenschaltung aus einer zweiten Sensorspule mit einem zweiten Widerstand gebildet. In dieser Ausführungsform wird ein Positionssignal durch einen Differenzverstärker als Differenzspannung der über dem ersten Widerstand und der zweiten Sensorspule abfallenden Spannungen oder als Differenz der Phasenlage der Spannungen gebildet.

Alternativ wird die Brückenschaltung durch die Parallelschaltung einer ersten Reihenschaltung aus einer ersten Sensorspule mit einem ersten Widerstand und einer zweiten Reihenschaltung aus einem zweiten Widerstand mit einer zweiten Sensorspule gebildet. In dieser Ausführungsform wird ein Positionssignal durch einen Differenzverstärker als Differenzspannung der über dem ersten Widerstand und dem zweiten Widerstand abfallenden Spannungen oder als Differenz der Phasenlage der Spannungen gebildet.

In allen Ausführungsformen kann das Positionssignal durch eine Gleichrichterschaltung gleichgerichtet werden. Vor der Gleichrichtung kann das Positionssignal durch ein Bandpassfilter mit einer der Frequenz des Wechselstroms entsprechenden Mittenfrequenz gefiltert werden.

Die Sensorspulen sind vorteilhafterweise zumindest einer der Stirnseiten des Bauteils gegenüberliegend angeordnet, wobei die Stirnseiten des Bauteils zumindest teilweise aus einem leitfähigen Material bestehen oder Körper aus einem leitfähigen Material enthalten. Das leitfähige Material ist bevorzugt ein Nichteisenmetall, wie Aluminium oder Kupfer, die den Vorteil einer sehr guten Leitfähigkeit bei geringem Gewicht haben.

Wenn die Frequenz des die Sensorspulen durchfließenden Wechselstroms der Resonanzfrequenz eines Resonanzkreises entspricht, der durch die Induktivität einer Sensorspule und einer Kapazität gebildet wird, kann der Realteil des Wechselstromwiderstandes der Sensorspule mit einer Brückenschaltung besonders einfach bestimmt werden.

Die Kapazität des Resonanzkreises kann durch die Wicklungskapazität einer Sensorspule oder durch einen Kondensator gebildet werden.

Besonders vorteilhaft ist es, wenn die magnetischen Felder der Sensorspulen durch das bewegliche Bauteil gegenläufig beeinflusst werden. In dieser Ausführungsform haben temperaturbedingte Veränderungen des Wechselstromwiderstandes der Sensorspulen keinen Einfluss auf das Positionssignal.

Das Verfahren zur Positionsbestimmung eines zumindest in einer Richtungsachse beweglichen Bauteils einer Blutpumpe mit zumindest einer von einem Wechselstrom durchflossenen Sensorspule, deren magnetisches Feld durch das bewegliche Bauteil beeinflusst wird, wobei die Sensorspule Bestandteil eines Resonanzkreises ist, umfasst die Bestimmung der Position des beweglichen Bauteils durch die Auswertung des Wechselstromwiderstandes oder der Resonanzfrequenz des Resonanzkreises.

Die Erfindung wird nachstehend anhand von Ausführungsbeispielen näher erläutert. Die beigefügten Zeichnungen stellen dar:
- Fig. 1: eine symbolische Darstellung der Wirbelstrombildung,
- Fig. 2: einen Querschnitt durch eine Fluidförderpumpe,
- Fig. 3A - 3C: die Sensoranordnungen,
- Fig. 4A - 4C: eine schematische Darstellung verschiedener Bauteilpositionen in einer Richtungsachse,
- Fig. 5: das Ersatzschaltbild der Sensorspulen,
- Fig. 6: eine Widerstandsbrückenschaltung,
- Fig. 7A - 7C: die Schaltungsvarianten einer Brückenschaltung sowie
- Fig. 8: die Auswerteschaltung.

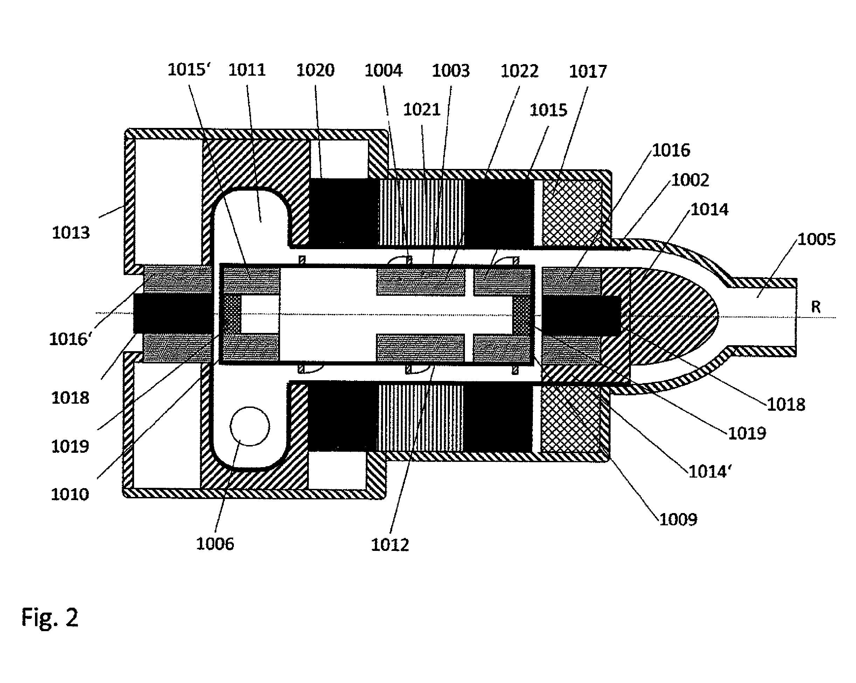

In Fig. 2 ist ein Längsschnitt durch eine als Blutpumpe 1001 ausgebildete Fluidförderpumpe eingangs beschriebener Art schematisch dargestellt. Die Blutpumpe 1001 umfasst einen Hohlkörper 1002 (als durchgezogene dicke Linie dargestellt), in dem ein in der Rotationsachse R (gestrichelt dargestellt) rotierendes Laufrad 1003 mit einer Beschaufelung 1004 vorgesehen ist. Ferner weist der Hohlkörper 1002 einen Einlass 1005 zum Einströmen von Blut in einer zur Rotationsachse R parallelen Einströmrichtung und einen Auslass 1006 zum Ausströmen des Blutes in einer Ausströmrichtung, welche senkrecht zur Schnittebene verläuft, auf.

Der Auslass 1006 des Hohlkörpers 1002 ist zwischen einer dem Einlass zugewandten Zuströmseite 1009 des Laufrades 1003 und einer dem Einlass abgewandten Abströmseite 1010 des Laufrads 1003 angeordnet. Ein Innenradius des Hohlkörpers 1002 dient zur Ausbildung eines das Laufrad 1003 tangential umlaufenden und in den Auslass 1006 mündenden Abflusskanals 1011 für ein im Wesentlichen tangential zum Laufrad 1003 verlaufendes Abfließen des Blutes aus dem Hohlkörper 1002.

Eine die Beschaufelung 1004 tragende Mantelfläche 1012 des Laufrads 1003 ist zylinderförmig ausgeformt, könnte aber genauso gut kegelstumpfförmig oder kegelförmig ausgestaltet sein. Eine axiale Abmessung (Länge) des Laufrads 1003 ist größer gewählt als ein Durchmesser des Laufrads 1003 auf der Abströmseite 1010 des Laufrads.

Die Beschaufelung 1004 des Laufrads 1003 ist durch eine Steigung gekennzeichnet. Die Steigung kann gleichmäßig sein oder zum Auslass 1006 hin zunehmen. Auf diese Weise wird ein für Blut besonders schonender axialer Vortrieb zum Abflusskanal 1011 hin ermöglicht.

Die Beschaufelung 1004 kann sich vollständig oder teilweise über die Mantelfläche 1012 erstrecken. Der in den Ausflusskanal 1011 ragende Teil des Laufrads 1003 kann dabei von der Beschaufelung ausgenommen werden.

In unmittelbarer Nähe zur Zuströmseite 1009 des Laufrads 1003 ist ein Vorleitrad 1014 vorgesehen, welches mit einer Beschaufelung 1014' ausgestattet ist.

Die Blutpumpe umfasst ferner eine teilweise aktiv stabilisierte Lagerungsvorrichtung, welche eine aktiv stabilisierte magnetische Axiallagerung sowie eine passive magnetische Radiallagerung beinhaltet.

Die magnetische Lagerungsvorrichtung umfasst zunächst zwei in dem Laufrad 1003 auf der Zuströmseite 1009 und an der Abströmseite 1010 angeordnete Permanentmagnete 1015, 1015'. Ferner dienen zwei weitere Lagermagnete 1016, 1016' der Ausbildung des passiven magnetischen Radiallagers, welches dafür sorgt, dass das Laufrad 1003 in einer radialen Sollposition zwischen dem Vorleitrad 1014 und der Rückabschlussplatte 1013 gehalten wird. Die Paare der Permanentmagnete 1015, 1016; 1015', 1016' sind auf Anziehung gepolt. Magnetisch liegen die Paare in Reihe.

In radialer Richtung wirken beide Magnetpaare zentrierend, die radiale Lage des Laufrads 1003 ist somit passiv stabil. Ohne eine zusätzliche Stabilisierung würde das Laufrad 1003 zur Einströmseite 1009 oder zur Ausströmseite 1010 hin angezogen werden, es besteht in axialer Richtung ein instabiles Gleichgewicht.

Für die aktive Stabilisierung der magnetischen Axiallagerung ist eine Ringspule 1017 außerhalb des Hohlkörpers 1002 vor dem Laufrad 1003 so angeordnet, dass sie den Hohlkörper 1002 ringförmig zur Erzeugung eines axialen Magnetflusses umläuft.

Ein Stromfluss durch die Ringspule 1017 erzeugt einen Magnetfluss, welcher das Magnetfeld des Magnetpaares 1015, 1016 beeinflusst und somit eine Regelung der axialen Lage des Laufrades 1003 ermöglicht. Der Stromfluss durch die Ringspule 1017 wird in Abhängigkeit von der Position des Laufrades 1003 in der axialen Richtungsachse geregelt.

Zur Bestimmung der Position des Laufrades 1003 in der axialen Richtungsachse ist zumindest eine Sensorspule 1018 der Einströmseite 1009 und/oder der Ausströmseite 1010 des Laufrades 1003 gegenüberliegend angeordnet. Das Laufrad 1003 weist auf der der Sensorspulen 1018 gegenüberliegenden Seite einen Metallkörper 1019 auf.

Das Laufrad 1003 bildet den Rotor eines bürstenlosen Synchronmotors. Der Stator dieses Motors wird durch eine den Hohlkörper 1002 umlaufende Motorwicklung 1020, ein Blechpaket 1021 und in der Figur nicht detailliert dargestellte Eisenabschlusskappen gebildet. Weiterhin ist zum Antreiben des Laufrads 1003 ein im Laufrad 1003 integrierter Motormagnet 1022 vorgesehen, welcher alternierend radial magnetisiert ist.

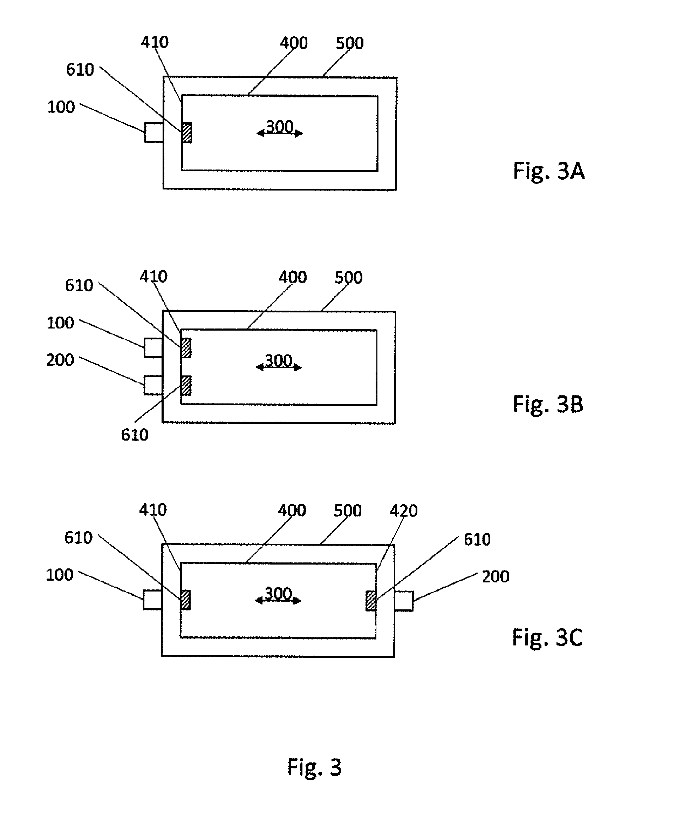

In den Figuren 3A bis 3C sind verschiedene Lagesensoranordnungen mit einer oder mehreren Sensorspulen dargestellt. Ein Bauteil 400 ist innerhalb einer Umhüllung 500 zumindest in einer Richtungsachse 300 beweglich angeordnet. Das Bauteil 400 wird in der Richtungsachse 300 durch die Stirnseiten 410 und 420 begrenzt. Zur Bestimmung der Position des Bauteils 400 in der Richtungsachse 300 sind gegenüber einer oder mehrerer der Stirnseiten 410 bzw. 420 des Bauteils 400 eine oder mehrere Sensorspulen 100 bzw. 200 angeordnet. Das Bauteil 400 kann beispielsweise durch den bereits erwähnten magnetisch, zum Beispiel permanentmagnetisch gelagerten Rotor eines bürstenlosen Synchronmotors, wie das in Fig. 2 dargestellte Laufrad 1003, gebildet werden.

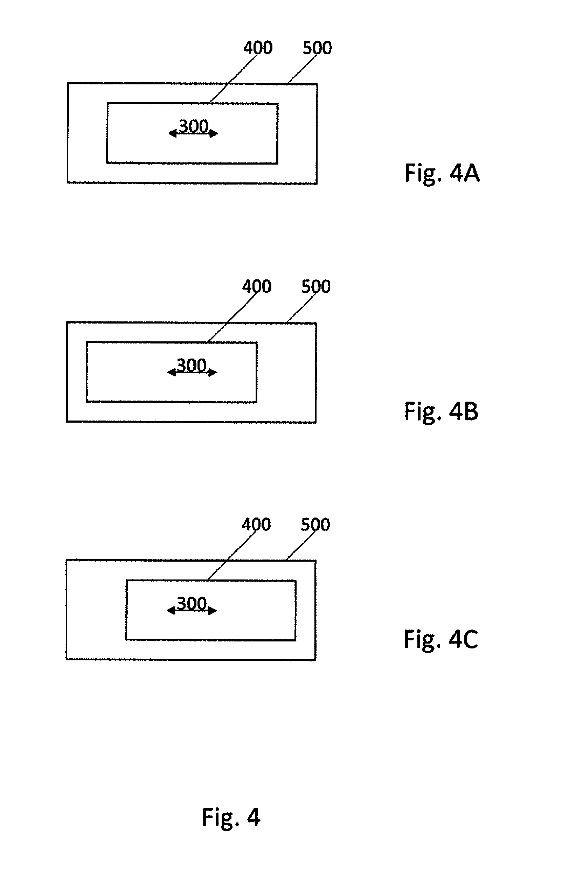

In den Figuren 4A bis 4C sind schematisch verschiedene Positionen des Bauteils 400 in der Richtungsachse 300 dargestellt. Fig. 4A zeigt das Bauteil 400 in einer Mittenposition, während Fig. 4B und Fig. 4C das Bauteil 400 in der Richtungsachse 300 nach links beziehungsweise nach rechts verschoben darstellen. Die in Fig. 4B und Fig. 4C dargestellten Positionen des Bauteils 400 illustrieren beispielhaft mögliche Endpunkte für die Bewegungen des Bauteils 400 in der Richtungsachse 300 und damit den Messbereich der Lagesensoranordnung.

Bei den in Fig. 3 dargestellten Lagesensoranordnungen besteht zumindest ein Teil der Stirnseite des Bauteils 400, welches der Sensorspule 100 oder 200 gegenüberliegt, aus einem metallischen Material, in dem sich Wirbelströme bilden, wenn die Sensorspule von einem Wechselstrom durchflossen wird. Die Stärke der Wirbelstrombildung ist abhängig von der Entfernung zur Sensorspule und beeinflusst das Magnetfeld der Sensorspule. Durch die abstandsabhängige Veränderung des Magnetfeldes der Sensorspule verändert sich auch deren Wechselstromwiderstand. Es kann an der entsprechenden Stirnseite des Bauteils 400 auch ein Metallkörper 610 vorgesehen werden, wie er in Fig. 3 gezeigt ist. Das metallische Material der Stirnseite des Bauteils 400 oder des Metallkörpers 610 ist bevorzugt ein Nichteisenmetall, beispielsweise Aluminium oder Kupfer.

Fig. 3A zeigt eine aus dem Stand der Technik bekannte erste Lagesensoranordnung zur Positionsbestimmung eines Bauteils 400 mittels einer Sensorspule. Zur Bestimmung der Position des Bauteils 400 in der Richtungsachse 300 ist gegenüber einer Stirnseite 410 des Bauteils 400 eine Sensorspule 100 angeordnet.

Bei der in Fig. 3A gezeigten Lagesensoranordnung vergrößert sich der Wechselstromwiderstand der Sensorspule bei einer Annäherung des Bauteils 400 an die Sensorspule 100 in der Richtungsachse 300 aufgrund der vergrößerten Wirbelstrombildung, während sich der Wechselstromwiderstand der Sensorspule 100 bei einer Entfernung des Bauteils 400 von der Sensorspule 100 in der Richtungsachse 300 aufgrund der verringerten Wirbelstrombildung verkleinert. Aus dem Wechselstromwiderstand der Sensorspule 100 kann ein Maß für die Position des Bauteils 400 in der Richtungsachse 300 abgeleitet werden. Ebenso kann aus der Änderung des Wechselstromwiderstandes der Sensorspule 100 in Folge einer Verschiebung des Bauteils 300 in der Richtungsachse 300 ein Maß für die Änderung der Position des Bauteils 400 in der Richtungsachse 300 abgeleitet werden.

Fig. 3B zeigt eine zweite anmeldungsgemäße Lagesensoranordnung zur Positionsbestimmung eines Bauteils 400 mit zwei Sensorspulen. Zusätzlich zu der in Fig. 3A gezeigten Sensorspule 100 ist eine zweite Sensorspule 200 gegenüber der Stirnseite 410 des Bauteils 400 angeordnet.

Bei der in Fig. 3B gezeigten Lagesensoranordnung ist die Beeinflussung der magnetischen Felder der Sensorspulen durch das Bauteil 400 und damit die Änderung des Wechselstromwiderstandes der Sensorspulen gleichläufig, d. h., der Wechselstromwiderstand beider Sensorspulen vergrößert sich bei einer Annäherung des Bauteils 400 in der Richtungsachse 300 an die Sensorspulen und verkleinert sich, wenn sich das Bauteil 400 in der Richtungsachse 300 von den Sensorspulen entfernt.

Fig. 3C zeigt eine dritte anmeldungsgemäße Lagesensoranordnung zur Positionsbestimmung eines Bauteils 400 mit zwei Sensorspulen. Zusätzlich zu der in Fig. 3A gezeigten Sensorspule 100 ist eine zweite Sensorspule 200 gegenüber der Stirnseite 420 des Bauteils 400 angeordnet.

Bei der in Fig. 3C gezeigten Lagesensoranordnung ist die Beeinflussung der magnetischen Felder der Sensorspulen durch das Bauteil 400 und damit die Änderung des Wechselstromwiderstandes der Sensorspulen gegenläufig. Wenn sich das Bauteil 400 in der Richtungsachse 300 an die erste Sensorspule 100 annähert, vergrößert sich der Wechselstromwiderstand der ersten Sensorspule 100, gleichzeitig entfernt sich das Bauteil 400 in der Richtungsachse 300 von der zweiten Sensorspule 200, wodurch sich der Wechselstromwiderstand der zweiten Sensorspule 200 verkleinert.

Bei den in Fig. 3B und Fig. 3C dargestellten Lagesensoranordnungen mit zwei Sensorspulen werden beide Sensorspulen gleichermaßen durch Änderungen der Temperatur beeinflusst.

Die Sensorspulen 100 und 200 weisen neben ihrer Induktivität aufgrund parasitärer Effekte auch einen ohmschen Widerstand und einen durch die Wicklungskapazität gebildeten kapazitiven Anteil auf. Der Wechselstromwiderstand einer Spule ist damit komplex und besteht aus einem Realteil, der im Wesentlichen den rein ohmschen Widerstand beschreibt, und aus einem Imaginärteil, der die induktiven und kapazitiven Anteile beinhaltet.

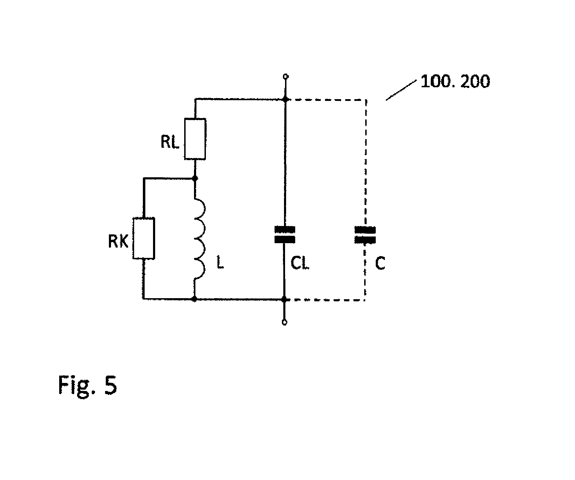

Fig. 5 zeigt das Ersatzschaltbild der Sensorspulen 100 beziehungsweise 200 mit der Induktivität L und der Wicklungskapazität CL sowie dem Widerstand der Sensorspulenwicklung RL und dem Kernwiderstand RK.

Durch den Kernwiderstand RK wird der bei einer Spule auftretende Effekt berücksichtigt, dass der Realteil des Wechselstromwiderstandes einer Spule größer ist als der mit einer Gleichstrommessung bestimmte ohmsche Widerstand RL. Der Kernwiderstand RK ist das Maß für die in den Realteil des Wechselstromwiderstandes eingehenden zusätzlichen Verluste im Wechselstrombetrieb, die beispielsweise durch Wirbelströme verursacht werden.

Wie aus Fig. 5 ersichtlich, kann die Sensorspule als verlustbehafteter Parallelresonanzkreis betrachtet werden. Die Resonanzfrequenz dieses Parallelresonanzkreises ergibt sich aus der Induktivität der Spule L und der Wicklungskapazität CL. Optional kann die Resonanzfrequenz durch Parallelschaltung eines Kondensators C geeigneter Kapazität auf einen beliebigen Wert eingestellt werden.

Wird die Sensorspule von einem Wechselstrom durchflossen, dessen Frequenz der Resonanzfrequenz des durch die Induktivität der Sensorspule, die Wicklungskapazität der Sensorspule und gegebenenfalls eine weitere Kapazität gebildeten Parallelresonanzkreises entspricht, heben sich die induktiven und kapazitiven Anteile des komplexen Wechselstromwiderstandes im Parallelresonanzkreis gegenseitig auf. Damit ist der Wechselstromwiderstand des Parallelresonanzkreises nur noch vom Widerstand der Spulenwicklung RL und dem durch die Wirbelstrombildung beeinflussten Kernwiderstand RK abhängig. Auf diese Weise kann der Realteil des Wechselstromwiderstandes einer Sensorspule durch eine Messung des ohmschen Widerstandes der Sensorspule bestimmt werden.

Die Wahl der Frequenz der die Sensorspule durchfließenden Wechselspannung als eine Frequenz, die der Resonanzfrequenz des durch die Induktivität der Sensorspule, die Wicklungskapazität der Sensorspule und gegebenenfalls eine weitere Kapazität gebildeten Paralleiresonanzkreises entspricht, ermöglicht eine wesentlich vereinfachte Messvorrichtung für die Bestimmung des Wechselstromwiderstandes der Sensorspule, da die ansonsten notwendige Kompensation für die induktiven und kapazitiven Anteile des komplexen Wechselstromwiderstandes der Sensorspule entfallen kann.

Eine Möglichkeit dazu ist eine Brückenschaltung.

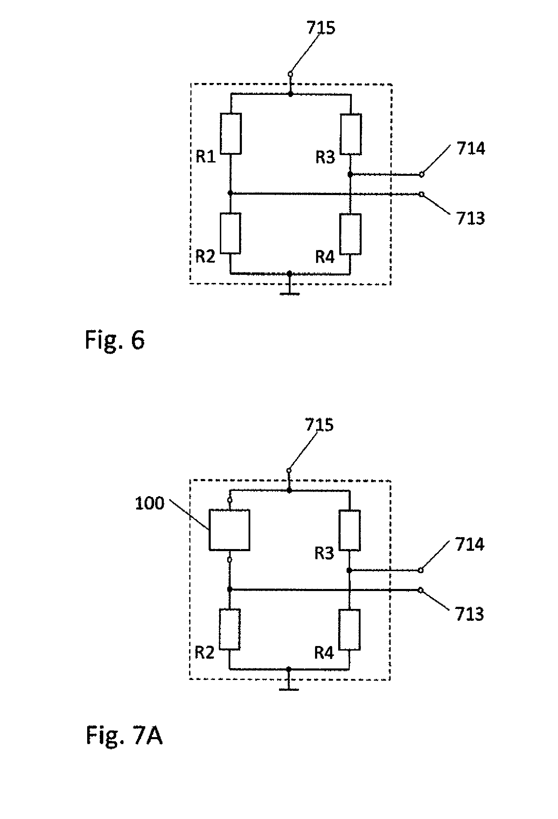

Fig. 6 zeigt eine Brückenschaltung 710 mit den Widerständen R1 bis R4. Die Widerstände R1 und R2 sind in Reihe geschaltet und bilden den ersten Brückenzweig. Die Widerstände R3 und R4 sind ebenfalls in Reihe geschaltet und bilden den zweiten Brückenzweig. Beide Brückenzweige sind an den Punkten 715 und Masse parallel geschaltet und werden zwischen den Punkten 715 und Masse mit einer Spannung beaufschlagt. An den Punkten 713 und 714 kann die so genannte Querspannung oder Brückenspannung gemessen werden. Wie aus Fig. 6 ersichtlich, bilden die beiden Brückenzweige mit den Widerständen R1 und R2 beziehungsweise R3 und R4 jeweils einen Spannungsteiler. Die Brückenspannung ist abhäng von den Widerstandsverhältnissen R1/R2 und R3/R4. Ist beispielsweise das Verhältnis R1/R2 = R3/R4, dann ist die Brückenspannung null.

Fig. 7A zeigt die Ausführung der Brückenschaltung 710 für die in Fig. 3A dargestellte Lagesensoranordnung. Die Reihenschaltung der Sensorspule 100 mit dem Widerstand R2 bildet den ersten Brückenzweig. Die Widerstände R3 und R4 sind ebenfalls in Reihe geschaltet und bilden den zweiten Brückenzweig. Beide Brückenzweige sind an den Punkten 715 und Masse parallel geschaltet und werden zwischen den Punkten 715 und Masse mit einer Wechselspannung beaufschlagt, dessen Frequenz der Resonanzfrequenz des durch die Induktivität der Sensorspule, die Wicklungskapazität der Sensorspule und gegebenenfalls eine weitere Kapazität gebildeten Parallelresonanzkreises entspricht. An den Punkten 713 und 714 wird die Brückenspannung zur weiteren Auswertung abgegriffen.

Bei entsprechender Wahl der Werte der Widerstände R2, R3 und R4 kann die Brückenschaltung so dimensioniert werden, dass die Brückenspannung bei einer der in Fig. 4 dargestellten Positionen des Bauteils 400 gleich null ist. Soll zum Beispiel die Brückenspannung in der in Fig. 4C dargestellten Position des Bauteils 400 den Wert Null annehmen, wird für R2, R3 und R4 der Wert des Wechselstromwiderstandes der Sensorspule 100 in dieser Position gewählt. Bei einer Verschiebung des Bauteils 400 auf der Richtungsachse 300 in Richtung zu der in Fig. 4B dargestellten Position vergrößert sich der Wechselstromwiderstand der Sensorspule 100 mit der Folge, dass aufgrund des geänderten Widerstandsverhältnisses von Sensorspule 100 und R2 die Brückenspannung zwischen den Punkten 713 und 714 einen Wert ungleich null annimmt, wobei der Betrag der Brückenspannung von der Position des Bauteils 400 auf der Richtungsachse abhängig ist.

Nachteilig bei der Lagesensoranordnung gemäß Fig. 3A ist unter anderem die Temperaturabhängigkeit des Wechselstromwiderstandes der Sensorspule 100, weil eine durch eine Temperaturänderung verursachte Änderung des Wechselstromwiderstandes der Sensorspule 100 die Brückenspannung direkt beeinflusst und das daraus gewonnene Positionssignal verfälscht.

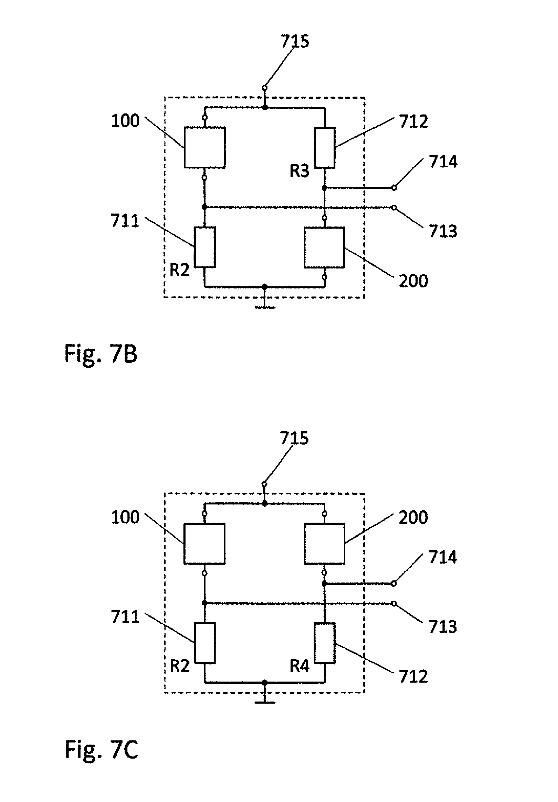

Fig. 7B zeigt die Ausführung der Brückenschaltung 710 für die in Fig. 3B dargestellte Lagesensoranordnung. Diese Brückenschaltung berücksichtigt die gleichläufige Änderung der Wechselstromwiderstände beider Sensorspulen, wobei durch deren kombinierte Auswertung in einer Brückenschaltung eine höhere Messgenauigkeit erreicht wird. Die Reihenschaltung der Sensorspule 100 mit dem Widerstand R2 bildet den ersten Brückenzweig. Der zweite Brückenzweig wird durch die Reihenschaltung des Widerstandes R3 mit der Sensorspule 200 gebildet. Diese Auslegung der Brückenschaltung berücksichtigt die gleichläufige Änderung der Wechselstromwiderstände beider Sensorspulen, um mit deren gleichzeitiger Auswertung eine höhere Messgenauigkeit zu erreichen. Beide Brückenzweige sind an den Punkten 715 und Masse parallel geschaltet und werden zwischen den Punkten 715 und Masse mit einer Wechselspannung beaufschlagt, dessen Frequenz der Resonanzfrequenz des durch die Induktivität der Sensorspule, die Wicklungskapazität der Sensorspule und gegebenenfalls eine weitere Kapazität gebildeten Parallelresonanzkreises entspricht. An den Punkten 713 und 714 wird die Brückenspannung zur weiteren Auswertung abgegriffen.

Analog zu der in Fig. 7A dargestellten Brückenschaltung kann auch die in Fig. 7B gezeigte Brückenschaltung durch eine entsprechende Wahl der Werte der Widerstände R2 und R3 so dimensioniert werden, dass die Brückenspannung bei einer der in Fig. 4 dargestellten Positionen des Bauteils 400 gleich null ist. Soll zum Beispiel die Brückenspannung in der in Fig. 4C dargestellten Position des Bauteils 400 den Wert null annehmen, wird für R2 der Wert des Wechselstromwiderstandes der Sensorspule 100 in dieser Position gewählt, während für R3 der Wert des Wechselstromwiderstandes der Sensorspule 200 in dieser Position gewählt wird. Bei einer Verschiebung des Bauteils 400 auf der Richtungsachse 300 in Richtung zu der in Fig. 4B dargestellten Position vergrößern sich die Wechselstromwiderstände beider Sensorspulen 100 und 200 mit der Folge, dass aufgrund des geänderten Widerstandsverhältnisses von Sensorspule 100 und R2 beziehungsweise Sensorspule 200 und R3 die Brückenspannung zwischen den Punkten 713 und 714 einen Wert ungleich null annimmt, wobei der Betrag der Brückenspannung von der Position des Bauteils 400 auf der Richtungsachse abhängig ist. Im Vergleich zu der in Fig. 7A dargestellten Brückenschaltung verdoppelt sich der Betrag der Brückenspannung bei einer ansonsten gleich großen Verschiebung des Bauteils 400, da durch die Brückenschaltung die Änderungen der Wechselstromwiderstände der Sensorspulen summiert werden, wodurch eine Verdoppelung der Messgenauigkeit erreicht wird.

Fig. 7C zeigt die Ausführung der Brückenschaltung 710 für die in Fig. 3C dargestellte Lagesensoranordnung. Diese Brückenschaltung berücksichtigt die gegenläufige Änderung der Wechselstromwiderstände beider Sensorspulen, wobei durch deren kombinierte Auswertung in einer Brückenschaltung eine höhere Messgenauigkeit erreicht wird. Die Reihenschaltung der Sensorspule 100 mit dem Widerstand R2 bildet den ersten Brückenzweig. Der zweite Brückenzweig wird durch die Reihenschaltung der Sensorspule 200 mit dem Widerstand R4 gebildet. Beide Brückenzweige sind an den Punkten 715 und Masse parallel geschaltet und werden zwischen den Punkten 715 und Masse mit einer Wechselspannung beaufschlagt, dessen Frequenz der Resonanzfrequenz des durch die Induktivität der Sensorspule, die Wicklungskapazität der Sensorspule und gegebenenfalls eine weitere Kapazität gebildeten Parallelresonanzkreises entspricht. An den Punkten 713 und 714 wird die Brückenspannung zur weiteren Auswertung abgegriffen.

Analog zu der in Fig. 7B dargestellten Brückenschaltung kann auch die in Fig. 7C gezeigte Brückenschaltung durch eine entsprechende Wahl der Werte der Widerstände R2 und R4 so dimensioniert werden, dass die Brückenspannung bei einer der in Fig. 4 dargestellten Positionen des Bauteils 400 gleich null ist. Soll zum Beispiel die Brückenspannung in der in Fig. 4C dargestellten Position des Bauteils 400 den Wert null annehmen, wird für R2 der Wert des Wechselstromwiderstandes der Sensorspule 100 in dieser Position gewählt, während für R4 der Wert des Wechselstromwiderstandes der Sensorspule 200 in dieser Position gewählt wird. Bei einer Verschiebung des Bauteils 400 in der Richtungsachse 300 in die in Fig. 4B dargestellte Position vergrößert sich der Wechselstromwiderstand der Sensorspule 100, während sich der Wechselstromwiderstand der Sensorspule 200 verkleinert. Aufgrund der geänderten Widerstandsverhältnisse von Sensorspule 100 und R2 beziehungsweise Sensorspule 200 und R4 nimmt die Brückenspannung zwischen den Punkten 713 und 714 einen Wert ungleich null an, wobei der Betrag der Brückenspannung von der Position des Bauteils 400 auf der Richtungsachse abhängig ist. Auch bei dieser Brückenschaltung verdoppelt sich im Vergleich zu der in Fig. 7A dargestellten Brückenschaltung der Betrag der Brückenspannung bei einer ansonsten gleich großen Verschiebung des Bauteils 400, da durch die Brückenschaltung die Änderungen der Wechselstromwiderstände der Sensorspulen summiert werden, wodurch ebenfalls eine Verdoppelung der Messgenauigkeit erreicht wird.

In dieser Brückenschaltung wird zusätzlich eine vollständige Temperaturkompensation erreicht. Da sich bei Temperaturänderungen die Wechselstromwiderstände beider Sensorspulen gleichermaßen verändern, wird die Brückenspannung durch temperaturbedingte Änderungen der Wechselstromwiderstände der Sensorspulen nicht beeinflusst.

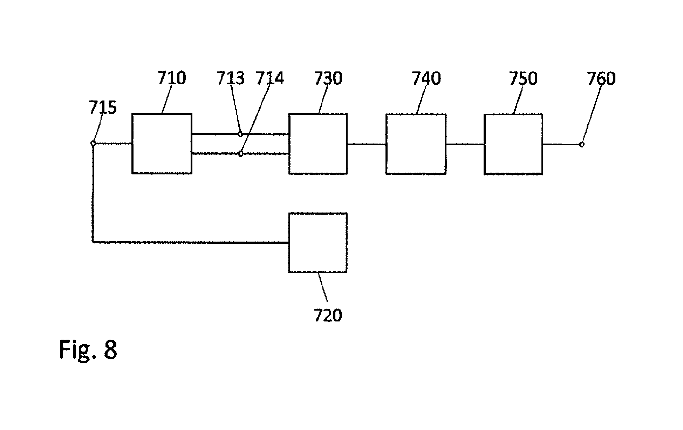

Fig. 8 zeigt das Blockschaltbild einer Schaltung 700 zur Bestimmung eines Positionssignals für die Lagesensoranordnungen gemäß Fig. 3. Die Sensorspule 100 beziehungsweise die Sensorspulen 100 und 200 sind Bestandteil der vorgehend ausführlich beschriebenen Brückenschaltung 710. Ein Wechselspannungsgenerator 720 führt der Brückenschaltung 710 über den Punkt 715 und Masse eine Wechselspannung zu, deren Frequenz der Resonanzfrequenz der durch die Induktivitäten der Sensorspulen, die Wicklungskapazitäten der Sensorspulen und gegebenenfalls weitere Kapazitäten gebildeten Parallelresonanzkreise entspricht. Der Wechselspannungsgenerator kann auch ausgebildet sein, eine Wechselspannung als Überlagerung zweier Wechselspannungen unterschiedlicher Frequenz abzugeben. Dies ist vorteilhaft, wenn die mit Sensorspulen 100 beziehungsweise 200 gebildeten Parallelresonanzkreise unterschiedliche Resonanzfrequenzen aufweisen. In diesem Fall kann jeder der Parallelresonanzkreise auf seiner Resonanzfrequenz betrieben werden. Die an den Punkten 713 und 714 gegen Masse anliegenden Spannungen werden an die Eingänge eines Differenzverstärkers 730 zugeführt. Das Ausgangssignal des Differenzverstärkers 730 ist die verstärkte Brückenspannung der Messbrücke 710.

Das Ausgangssignal des Differenzverstärkers 730 wird über ein optionales Bandpassfilter 740 einer Gleichrichterschaltung 750 zugeführt, an deren Ausgang 760 das gleichgerichtete und optional gefilterte Positionssignal anliegt.

Das optionale Bandpassfilter 740 dient der Verbesserung des Signal-Rausch-Verhältnisses des Positionssignals und hat bevorzugt eine Mittenfrequenz, die gleich der Frequenz der am Punkt 715 anliegenden Wechselspannung ist. Die Gleichrichterschaltung 750 kann beispielsweise als Hüllkurvendemodulator oder als Synchrondemodulator ausgeführt sein. Das Signal am Ausgang der Gleichrichterschaltung kann optional durch ein zusätzliches Tiefpassfilter geglättet werden.

Das Ausgangssignal des Differenzverstärkers 730 kann alternativ auch einem Analog/Digital-Wandler zur Umwandlung in ein digitalisiertes Signal zugeführt werden. Das Positionssignal kann dann durch weitere nachfolgende digitale Signalverarbeitung des digitalen Signals gewonnen werden.

Alternativ zur vorgehend beschriebenen Auswertung des Realteils des komplexen Wechselstromwiderstandes einer durch Wirbelstrombildung beeinflussten Sensorspule kann in den beschriebenen Lagesensoranordnungen auch der Imaginärteil des komplexen Wechselstromwiderstandes ausgewertet werden. In diesem Fall kann die Frequenz des die Sensorspule durchfließenden Wechselstroms so gewählt werden, dass sie nicht der Resonanzfrequenz des mit der Sensorspule gebildeten Resonanzkreises entspricht. Damit beeinflusst die Wirbelstrombildung auch die Induktivität der Sensorspule und bewirkt Veränderungen des Imaginärteils des komplexen Wechselstromwiderstandes der Sensorspule. Diese Änderungen können beispielsweise durch Auswertung der Phasenlage der über der Sensorspule abfallenden Wechselspannung bestimmt werden. Die Phasenänderungen können ebenfalls mit den in Fig. 7A - Fig. 7C dargestellten Brückenschaltungen ausgewertet werden. In dieser Ausführungsform wird die Differenz der Phasenlagen der an den Punkten 713 und 714 anliegenden Wechselspannungen bestimmt. Der Differenzverstärker 730 der in Fig. 8 gezeigten Auswerteschaltung ist dann ausgebildet, die Phasendifferenz der an den Punkten 713 und 714 anliegenden Wechselspannungen zu bestimmen.

Für die in Fig. 3A gezeigte Lagesensoranordnung kann das Positionssignal auch durch die Auswertung der Amplitude der über der Sensorspule abfallenden Wechselspannung bestimmt werden. In diesem Fall wird die Sensorspule von einem Wechselstrom durchflossen, dessen Frequenz der Resonanzfrequenz des durch die Induktivität der Sensorspule, die Wicklungskapazität der Sensorspule und gegebenenfalls eine weitere Kapazität gebildeten Resonanzkreises entspricht. Eine Lageänderung des Bauteils 400 verursacht, wie bereits beschrieben, eine Vergrößerung oder Verkleinerung der Wirbelstrombildung und beeinflusst damit auch die Induktivität der Sensorspule. Durch die Änderung der Induktivität der Sensorspule verändert sich die Resonanzfrequenz des Resonanzkreises und bewirkt eine Änderung des Wechselstromwiderstandes des Resonanzkreises. Diese, von der Lageänderung des Bauteils 400 abhängige Änderung verursacht eine entsprechende Änderung der Amplitude der über der Sensorspule abfallenden Wechselspannung, die mit der in Fig. 7A dargestellten Brückenschaltung und der in Fig. 8 gezeigten Auswerteschaltung als Positionssignal ausgewertet werden kann.

Die Auswertung der durch eine Lageänderung des Bauteils 400 verursachten Veränderung der Resonanzfrequenz eines mit der Sensorspule gebildeten Resonanzkreises kann vorteilhaft auch auf die in Fig. 3C gezeigte Anordnung mit zwei Sensorspulen angewendet werden. Die bereits beschriebene gegenläufige Beeinflussung der Sensorspulen in der in Fig. 3C gezeigten Anordnung verursacht eine gegenläufige Änderung der Resonanzfrequenz der mit den Sensorspulen gebildeten Resonanzkreise, das heißt, die Resonanzfrequenz eines der Resonanzkreise nimmt ab, während die Resonanzfrequenz des anderen Resonanzkreises zunimmt. Die Änderung der Resonanzfrequenzen bewirkt eine Phasenverschiebung der über den Sensorspulen abfallenden Wechselspannungen, aus der sich ebenfalls ein Positionssignal gewinnen lässt. Beispielsweise kann die Phasendifferenz der an den Sensorspulen abfallenden Wechselspannungen als Phasendifferenzsignal ermittelt werden. Weiterhin kann aus dem Phasendifferenzsignal, beispielsweise durch Vergleich mit der Speisespannung der Sensorspulen mittels eines XOR-Gliedes, ein pulsweitenmoduliertes (PWM-) Signal gewonnen werden, welches die Positionsänderung des Bauteils durch eine Änderung des Tastverhältnisses des PWM-Signals kodiert.

Wenn für die Gewinnung des Positionssignals Änderungen der Resonanzfrequenz eines mit einer Sensorspule gebildeten Resonanzkreises ausgewertet werden, ist es vorteilhaft, dass die Resonanzkreise eine hohe Kreisgüte aufweisen. Wenn für die Gewinnung des Positionssignals Änderungen des Realteiles des Wechselstromwiderstandes eines mit einer Sensorspule gebildeten Resonanzkreises ausgewertet werden, ist es hingegen vorteilhaft, dass die Resonanzkreise eine niedrige Kreisgüte aufweisen.

## Patentansprüche

1. Vorrichtung zur Positionsbestimmung eines zumindest in einer Richtungsachse (300) beweglichen Bauteils (400) einer Blutpumpe mit zwei von einem Wechselstrom durchflossenen Sensorspulen (100, 200), deren magnetische Felder durch das bewegliche Bauteil (400) beeinflusst werden,
**dadurch gekennzeichnet,**
**dass** die Sensorspulen (100, 200) in einer Brückenschaltung angeordnet sind, die ausgebildet ist, ein von der Differenz der Wechselstromwiderstände der Sensorspulen (100, 200) abhängiges Positionssignal auszugeben.

2. Vorrichtung nach Anspruch 1,
**dadurch gekennzeichnet,**
**dass** die Brückenschaltung (710) ausgebildet ist, den Realteil oder den Imaginärteil des komplexen Wechselstromwiderstandes der Sensorspulen (100, 200) auszuwerten.

3. Vorrichtung nach Anspruch 1 oder 2,
**dadurch gekennzeichnet,**
**dass** die Brückenschaltung (710) durch die Parallelschaltung einer ersten Reihenschaltung aus einer ersten Sensorspule (100) mit einem ersten Widerstand (711) und einer zweiten Reihenschaltung aus einer zweiten Sensorspule (200) mit einem zweiten Widerstand (712) gebildet wird.

4. Vorrichtung nach Anspruch 1 oder 2,
**dadurch gekennzeichnet,**
**dass** die Brückenschaltung (710) durch die Parallelschaltung einer ersten Reihenschaltung aus einer ersten Sensorspule (100) mit einem ersten Widerstand (711) und einer zweiten Reihenschaltung aus einem zweiten Widerstand (712) mit einer zweiten Sensorspule (200) gebildet wird.

5. Vorrichtung nach Anspruch 3,
**dadurch gekennzeichnet,**
**dass** ein Positionssignal durch einen Differenzverstärker (730) als Differenzspannung der über dem ersten Widerstand (711) und der zweiten Sensorspule (200) abfallenden Spannungen gebildet wird.

6. Vorrichtung nach Anspruch 4,
**dadurch gekennzeichnet,**
**dass** ein Positionssignal durch einen Differenzverstärker (730) als Differenzspannung der über dem ersten Widerstand (711) und dem zweiten Widerstand (712) abfallenden Spannungen gebildet wird.

7. Vorrichtung nach Anspruch 5 oder 6,
**dadurch gekennzeichnet,**
**dass** das Positionssignal durch eine Gleichrichterschaltung (750) gleichgerichtet wird.

8. Vorrichtung nach Anspruch 7,
**dadurch gekennzeichnet,**
**dass** das Positionssignal vor der Gleichrichtung durch ein Bandpassfilter (740) mit einer der Frequenz des Wechselstroms entsprechenden Mittenfrequenz gefiltert wird.

9. Vorrichtung nach einem der vorhergehenden Ansprüche,
**dadurch gekennzeichnet,**
**dass** die Sensorspulen (100, 200) zumindest einer der Stirnseiten (410, 420) das Bauteils (400) gegenüberliegend angeordnet sind.

10. Vorrichtung nach Anspruch 9,
**dadurch gekennzeichnet,**
**dass** die Stirnseiten (410, 420) des Bauteils (400) zumindest teilweise aus einem leitfähigen Material bestehen oder Körper (610, 620) aus einem leitfähigen Material enthalten.

11. Vorrichtung nach Anspruch 10,
**dadurch gekennzeichnet,**
**dass** das leitfähige Material ein Nichteisenmetall ist.

12. Vorrichtung nach einem der vorhergehenden Ansprüche,
**dadurch gekennzeichnet,**
**dass** die Frequenz des Wechselstroms der Resonanzfrequenz eines Resonanzkreises entspricht, der durch die Induktivität einer Sensorspule und einer Kapazität gebildet wird.

13. Vorrichtung nach Anspruch 12,
**dadurch gekennzeichnet,**
**dass** die Kapazität durch die Wicklungskapazität einer Sensorspule oder einen Kondensator gebildet wird.

14. Vorrichtung nach einem der vorhergehenden Ansprüche,
**dadurch gekennzeichnet,**
**dass** die magnetischen Felder der Sensorspulen (100, 200) durch das bewegliche Bauteil (400) gegenläufig beeinflusst werden.

15. Verfahren zur Positionsbestimmung eines zumindest in einer Richtungsachse (300) beweglichen Bauteils (400) einer Blutpumpe mit zumindest einer von einem Wechselstrom durchflossenen Sensorspule (100, 200), deren magnetisches Feld durch das bewegliche Bauteil (400) beeinflusst wird, wobei die Sensorspule (100, 200) Bestandteil eines Resonanzkreises ist;
**dadurch gekennzeichnet,**
**dass** die Position des beweglichen Bauteils (400) durch die Auswertung des Wechselstromwiderstandes oder der Resonanzfrequenz des Resonanzkreises bestimmt wird.
